# NOUVEAU FASCICULE DE BREVET EUROPEEN

(11) **EP 1 043 969 B2**
(45) Date de publication et mention de la décision concernant l'opposition: **12.01.2011**
(45) Mention de la délivrance du brevet: 06.06.2007
(21) Numéro de dépôt: 99950819.5
(22) Date de dépôt: 25.10.1999
(51) Int. Cl.: A61Q 5/06, A61K 8/90

(54) **DISPOSITIF FLACON POMPE CONTENANT UN POLYCONDENSAT COMPRENANT AU MOINS UN MOTIF POLYURETHANNE**
FLASCHENFÖRMIGER PUMPSPENDER ENTHALTEND EIN POLYKONDENSAT MIT MINDESTENS EINER POLYURETHANGRUPPIERUNG
AEROSOL DEVICE CONTAINING A CONDENSATION POLYMER COMPRISING AT LEAST A POLYURETHANE UNIT

(30) Priorité: 03.11.1998 FR 9813807
(43) Date de publication de la demande: 18.10.2000
(73) Titulaire: L'Oréal, 75008 Paris (FR)
(72) Inventeur: VILBERT, Arnaud, F-92390 Villeneuve-La-Garenne (FR)
(74) Mandataire: Bourdeau, Françoise
(86) Numéro de dépôt international: PCT/FR1999/002585
(87) Numéro de publication internationale: WO 2000/025736

(56) Documents cités:
- EP-A- 0 745 373
- EP-A- 0 838 212
- EP-A- 0 925 774
- WO-A1-91/10420
- WO-A1-97/17386
- DE-A- 4 438 846
- DE-A- 19 541 326
- DE-A1- 4 225 045
- US- - 5 686 067
- US-A- 5 643 581

## Description

L'invention a pour objet des dispositifs flacons pompes comprenant un réservoir qui contient, dans un milieu cosmétiquement acceptable, un polymère (A) multiséquencé comprenant au moins un motif polyuréthanne et un polymère filmogène (B), les polymères (A) et (B) et le dispositif étant choisis de sorte à obtenir, à la sortie du dispositif, des gouttelettes de composition de diamètre moyen inférieur à 80 µm. Elle vise également un procédé pour la mise en forme ou le maintien de la coiffure comprenant la mise en oeuvre de ces dispositifs ainsi que leur utilisation pour la fabrication d'un produit de coiffage.

La fixation de la coiffure est un élément important du coiffage qui consiste à maintenir la mise en forme déjà réalisée ou à mettre en forme les cheveux et à les fixer simultanément.

Les produits capillaires pour la mise en forme et/ou le maintien de la coiffure les plus répandus sur le marché de la cosmétique sont des compositions à pulvériser essentiellement constituées d'une solution le plus souvent alcoolique et/ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution peut être conditionnée par exemple dans un flacon pompe.

Pour satisfaire aux obligations liées à l'environnement, les compositions pour la fixation de la coiffure doivent dégager de moins en moins de composés organiques volatiles (VOC). A cet effet, la quantité de solvants organiques volatiles dans la composition est diminuée et ces solvants sont remplacés par de l'eau. Toutefois, cette modification de la formulation des compositions capillaires a pour effet néfaste d'augmenter fortement leur viscosité.

Le conditionnement sous forme flacon pompe est spécialement pratique pour l'utilisateur qui peut facilement doser la quantité de produit qu'il souhaite appliquer. Toutefois, ce type de conditionnement rend parfois difficile l'application du produit de façon homogène sur les cheveux, car les gouttelettes de produit sortant du flacon pompe sont souvent trop grosses. Cet inconvénient est particulièrement marqué pour les compositions visqueuses pour lesquelles des gouttelettes fines sont difficilement obtenues.

La qualité de la pulvérisation obtenue au moyen d'un dispositif flacon pompe, c'est-à-dire essentiellement la distribution des gouttelettes dans l'espace à la sortie de la buse, dépend fortement de la constitution chimique de la composition mise en oeuvre. On porte donc un intérêt tout particulier à la réalisation de dispositifs flacons pompes qui donnent lieu à de fines gouttelettes, en dépit d'une viscosité élevée des compositions.

Il est connu par le brevet DE 195 41 326 de préparer des compositions de coiffage comprenant un polymère à motif polyuréthanne en tant que polymère fixant. Les dispositifs peuvent toutefois être améliorés en ce qui concerne notamment les propriétés cosmétiques qu'ils confèrent aux cheveux tout en offrant une meilleure qualité de pulvérisation.

De manière surprenante et inattendue, la Demanderesse a découvert, contre toute attente, qu'il était possible de réaliser des dispositifs flacons pompes qui satisfont aux exigences exprimées ci-dessus, en opérant une sélection d'une part, sur la composition cosmétique et d'autre part, sur les moyens de distribution de cette composition.

L'invention a pour objet un dispositif flacon pompe comprenant un réservoir contenant une composition capillaire ainsi que des moyens de distribution de la composition, caractérisé par le fait que:
(1) la composition comprend, dans un milieu cosmétiquement acceptable, au moins un polycondensat (A) comprenant au moins une séquence polyuréthanne et au moins un polymère filmogène (B) différent de (A);
(2) les polymères (A) et (B) et le dispositif étant choisis de sorte à obtenir, à la sortie du dispositif, des gouttelettes de composition de diamètre moyen inférieur ou égal à 80 µm;
le polycondensat est formé par un arrangement de blocs, cet arrangement étant obtenu à partir de:
(1) au moins un composé qui contient deux ou plus de deux atomes d'hydro gène actifs par molécule;
(2) au moins un diol ou un mélange de diols contenant des radicaux acides ou leurs sels;
(3) au moins un di- ou polyisocyanate,
le polycondensat étant formés à partir de composés supplémentaires (5) ayant un squelette siliconé tels que les polysiloxanes, les polyalkylsiloxanes ou les polyarylsiloxanes notamment les polyéthylsiloxanes, les polyméthylsiloxanes et les polyphénylsiloxanes, comportant éventuellement des chaînes hydrocarbonées greffées sur les atomes de silicium.

Et les composés (1) sont choisis dans le groupe comprenant les polyesterols;

le diamètre des gouttelettes est mesuré pour une température de la composition voisine de 20°C à l'intérieur du dispositif flacon pompe et le diamètre des gouttelettes est mesuré à 20 cm de la buse.

Un autre objet de l'invention concerne un procédé pour la mise en forme ou le maintien de la coiffure comprenant la mise en oeuvre de ce dispositif flacon pompe.

Encore un autre objet de l'invention concerne l'utilisation de ce dispositif pour la fabrication d'un produit de coiffage.

Les polycondensats comprenant au moins une séquence polyuréthanne particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

Les polycondensats utilisés conformément à l'invention peuvent être solubles dans le milieu cosmétiquement acceptable, notamment après neutralisation par une base organique ou minérale, ou encore former une dispersion dans ce milieu. La dispersion peut comprendre alors au moins 0,05 % de tensioactif permettant la mise en dispersion et le maintien en dispersion du polycondensat.

Selon l'invention, on peut utiliser tout type de tensioactif dans ladite dispersion, mais de préférence un tensioactif non ionique. La taille moyenne des particules du polycondensat dans la dispersion est de préférence comprise entre 0,1 et 1 micron.

Les composés (1) sont choisis dans le groupe comprenant les polyesterols.

Parmi ceux-ci, on cite plus particulièrement les composés polyhydroxy tels que les polyester dols, les polyester polyamide diols.

Les polyesters diols sont de préférence saturés et sont obtenus, par exemple, à partir de la réaction d'acides polycarboxyliques saturés ou insaturés avec des alcools polyhydriques. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylène glycol, le 1,2-propylène glycol, le 1,4-butanediol, le néopentyl glycol et l'hexane diol.

Le composé du groupe (1) est un polyestérol, notamment un polyester diol formé par la réaction d'au moins un (di)-polyol (1ₐ) et d'au moins un acide (1_{b}). Le (di)- polyol (1ₐ) est en particulier choisi dans le groupe comprenant le néopentylglycol, le butanediol-1,4, l'hexanediol, l'éthylèneglycol, le diéthylène glycol, le propylèneglycol, le butylèneglycol, le néopentylglycol et (di)-polyéthylèneglycol. L'acide (1_{b}) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol propanoïque (DMPA) ou un acide carboxylique 2,2-hydroxyméthyl. En général, le composé (2) est utile en tant que bloc de couplage. En tant que composés (2), on préfère ceux comprenant au moins un poly (acide-(alpha-hydroxycarboxyliquediol)).

Les composés (2) particulièrement préférés conformément à l'invention sont ceux choisis dans le groupe comprenant le 2,2-di-(hydroxyméthyl) acide acétique, le 2,2-dihydroxyméthyl acide propionique, le 2,2-dihydroxyméthyl acide butyrique, l'acide 2,2-dihydroxyméthyl acide pentanoïque.

Le di- ou polyisocyanate (3) peut être choisi en particulier dans le groupe comprenant l'hexaméthylène diisocyanate, l'isophorondiisocyanate (IDPI), le toluylendiisocyanate, le diphénylméthane 4,4'-diisocyanate (DPMD) et le dicyclohexylméthane 4,4'-diisocyanate (DCMD), le méthylène-di-p-phényl diisocyanate, le méthylène-bis(4-cyclohexylisocyanate), l'isophorone diisocyanate, le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4-et de 2,6- toluène diisocyanate, le 2,2'-dichloro-4,4'-diisocyanato diphénylméthane, le 2,4-dibromo-1,5-diisocyanato naphtalène, le 1,4-diisocyanate butane, l'hexane-1,6-diisocyanate, le cyclohexane-1,4-diisocyanate.

Le polycondensat peut être formé à l'aide d'un composé supplémentaire (4) servant en général à allonger la chaîne du polycondensat. Ces composés (4) peuvent être choisis dans la groupe comprenant notamment les glycols saturés ou insaturés tel que l'éthylène glycol, le diéthylène glycol, le néopentylglycol, le triéthylène glycol, les aminoalcools tels que l'éthanolamine, la propanolamine, la butanolamine, les amines primaires hétérocyclique, aromatique, cycloaliphatique, et aliphatique, les diamines, les acides carboxylique tels que les acides carboxyliques aliphatique, aromatique, hétérocyclique comme l'acide oxalique, succinique, glutarique, adipique, sébacique, téréphtalique, les acides aminocarboxyliques. Les composés (4) préférés sont les diols aliphatiques.

Les séquences de polyuréthanne du polymère utilisées avantageusement présentent un motif répétitif de base répondant à la formule générale ci-après:

-X'-B-X'-CO-NH-R-NH-CO- (l')

dans laquelle :
- X' représente O
- B est un radical hydrocarboné bivalent, ce radical étant substitué ou non, et
- R est un radical divalent choisi parmi les radicaux alkylène de type aromatique, aliphatique en C₁ à C₂₀, cycloaliphatique en C₁ à C₂₀, ces radicaux étant substitués ou non.

De préférence, le radical B est un radical en C₁ à C₃₀ et est porteur d'un groupement présentant une ou des fonction(s) carboxylique(s) et/ou une ou des fonctions sulfoniques, lesdites fonctions carboxyliques et/ou sulfoniques étant sous forme libre ou bien neutralisées partiellement ou totalement par une base minérale ou organique.

Le radical R est avantageusement choisi parmi les radicaux répondant aux formules suivantes: 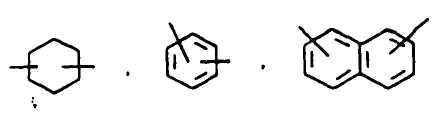 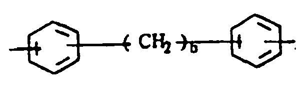 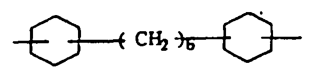 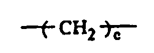 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4- et/ou 2,6-tolylène, 1,5-naphtylène, p-phénylène, méthylène- 4,4bis - cyclohexyle et le radical divalent dérivé de l'isophorone.

Le polycondensat mis en oeuvre conformément à l'invention comprenant au moins une séquence polyuréthanne peut avantageusement comprendre en outre au moins une séquence polysiloxane dont le motif répétitif de base répond par exemple à la formule générale (II') ci-après:

-X'-P-X'-CO-NH-R-NH-CO- (II')

dans laquelle :
- P est un segment polysiloxanique,
- X' représente O et
- R est un radical divalent choisi parmi les radicaux alkylènes de type aromatique, aliphatique en C₁ à C₂₀, cycloaliphatique en C₁ à C₂₀, ces radicaux étant substitués ou non.

Avantageusement, le segment polysiloxanique P répond à la formule générale ci-après: 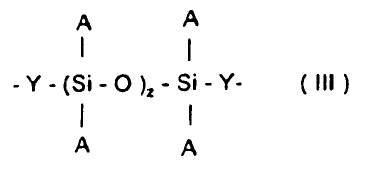 dans laquelle:
- les radicaux A, qui peuvent être identiques ou différents, sont choisis parmi d'une part les radicaux hydrocarbonés monovalents en C₁ à C₂₀ exempts ou substantiellement exempts d'insaturation éthylénique et, d'autre part, les radicaux aromatiques,
- Y représente un radical hydrocarboné bivalent, et
- z représente un nombre entier, choisi de telle sorte que le poids moléculaire moyen du segment polysiloxane soit compris entre 300 et 10 000.

En général, le radical bivalent Y est choisi parmi les radicaux alkylène de formule -(CH₂)ₐ-, dans laquelle a représente un nombre entier pouvant être compris entre 1 et 10.

Les radicaux A peuvent être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyle, en particulier le radical cyclohexyle, les radicaux aryle, notamment phényle et naphtyle, les radicaux arylalkyle, notamment benzyle et phényléthyle, ainsi que les radicaux tolyle et xylyle.

Les polymères filmogènes (B) cationiques, anioniques, amphotères et non ioniques utilisables conformément à l'invention sont décrits ci-après.

Les polymères filmogènes cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 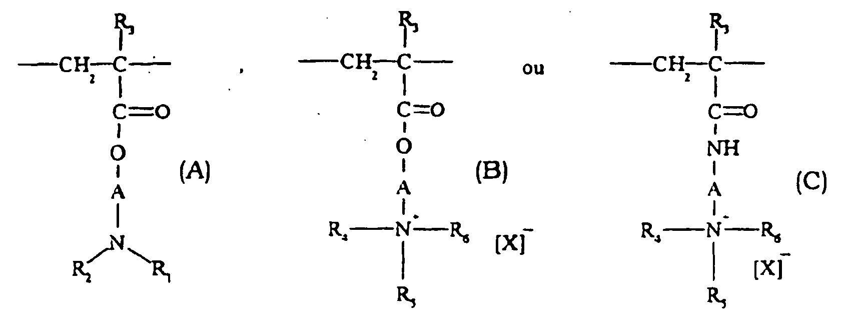 dans lesquelles:
   R₃ désigne un atome d'hydrogène ou un radical CH₃;
   A est un groupe alkyle linéaire ou ramifié de 1 à 6 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle;
   R₁ et R₂ représentent hydrogène ou un groupe alkyle ayant de 1 à 6 atomes de carbone;
   X désigne un anion méthosulfate ou un halogénure tel que chlorure ou bromure.
   Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous-la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactamel vinylpyrrolidone tel que le produit vendu sous la dénomination GAFFIX VC 713 par la société ISP,
   - et le copolymère vinylpyrrolidone / méthacrylamide de diméthylaminopropyle quaternisé tel que le produit vendu sous la dénomination "GAFQUAT HS 100" par la société ISP.
(2) les polysaccharides quaternisés décrits plus particulièrement dans les brevets américains 3.589.578 et 4.031.307 tel que les gommes de guar contenant des groupements cationiques trialkylammonium.
   De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole tels que les produits commercialisés par BASF sous l'appellation Luviquat TFC;
(4) les chitosanes ou leurs sels;
   les sels utilisables sont en particulier les acétate, lactate, glutamate, gluconate ou le pyrrolidone carboxylate de chitosane.
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone carboxylate de chitosane vendu sous la dénomination KYTAMER PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères filmogènes anioniques généralement utilisés sont des polymères comportant des groupements dérivés d'acide carboxylique, sulfonique ou phosphorique et ont un poids moléculaire compris entre environ 500 et 5.000.000.
1) Les groupements carboxyliques sont apportés par des monomères mono ou diacides carboxyliques insaturés tels que ceux répondant à la formule : 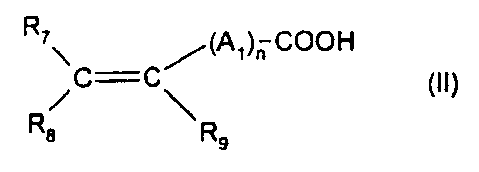 dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
   Dans la formule précitée un radical alkyle inférieur désigne de préférence un groupement ayant 1 à 4 atomes de carbone et en particulier, méthyle et éthyle.
   Les polymères filmogènes anioniques à groupements carboxyliques préférés selon l'invention sont :
   A) Les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
   B) Les copolymères des acides acrylique ou méthacrylique avec un monomère monoéthyténique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
   C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
   D) les copolymères dérivés d'acides ou d'anhydrides carboxyliques monoinsaturés en C₄-C₈ choisis parmi :
      - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US 2.047.398, 2.723:248, 2.102.113, le brevet GB 839.805 et notamment ceux vendus sous les dénominations GANTREZ AN ou ES par la société ISP.
      - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
         les fonctions anhydrides de ces copolymères étant éventuellement monoestérifiées ou monoamidifiées.
         Ces polymères sont par exemple décrits dans les brevets français 2.350.384 et 2.357.241 de la demanderesse.
   E) les polyacrylamides comportant des groupements carboxylates.
      Les polymères comprenant les groupements sulfoniques sont des polymères comportant des motifs vinylsulfonique, styrène sulfonique, naphtalène sulfonique ou acrylamido alkylsulfonique.
      Ces polymères peuvent être notamment choisis parmi:
      - les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
      - les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
      - les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.
      Selon l'invention, les polymères filmogènes anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique /acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.
      Les polymères filmogènes anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.
      Les polymères filmogènes amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;
      B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.
      Les polymères filmogènes amphotères répondant à la définition donnée ci-dessus plus particulièrement préférés sont choisis parmi les polymères suivants:

   1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
   (2) les polymères comportant des motifs dérivant :
      a) d'au moins un monomère choisi parmi les acrylamides ou les méthacrylamides substitués sur l'azote par un radical alkyle,
      b) d'au moins un comonomère acide contenant un ou plusieurs groupements carboxyliques réactifs, et
      c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
         Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
         Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
         Les comonomères basiques préférés sont des méthacrylates d'aminoéthyle, de butyl aminoéthyle, de N,N'-diméthylaminoéthyle, de N-tertio-butylaminoéthyle.
         On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
   (3) les polyamino amides réticulés et alcoylés partiellement ou totalement dérivant de polyaminoamides de formule générale : 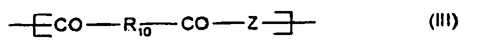 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
      a) dans les proportions de 60 à 100 moles %, le radical 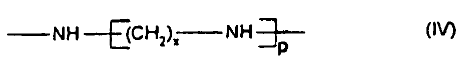 où x=2 et p=2 ou 3, ou bien x=3 et p=2
         ce radical dérivant de la diéthylène triamine, de la triéthylène tétraamine ou de la dipropylène triamine;
      b) dans les proportions de 0 à 40 moles % le radical (IV) ci-dessus; dans lequel x=2 et p=1 et qui dérive de l'éthylènediamine, ou le radical dérivant de la pipérazine : 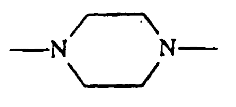
      c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
   (4) les polymères comportant des motifs zwittérioniques de formule: 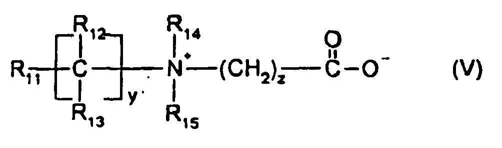 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
      Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
      A titre d'exemple, on peut citer le copolymère de méthacrylate de méthyle / diméthyl carboxyméthylammonio éthylméthacrylate de méthyle tel que le produit vendu sous la dénomination DIAFORMER Z301 par la société SANDOZ.
   (5) les polymères dérivés du chitosane comportant des motifs monomères répondant aux formules suivantes : 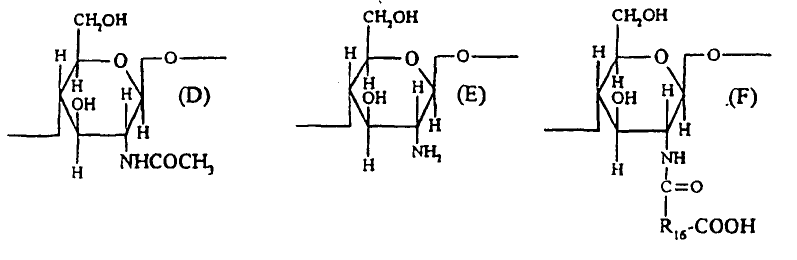 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 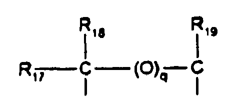 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R₁₇, R₁₈ et R₁₉ étant dans ce cas un atome d'hydrogène ;
      ou si q=1, R₁₇, R₁₈ et R₁₉ représentent chacun un atome d'hydrogène, ainsi que les sels formés par ces composés avec des bases ou des acides.
   (6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
   (7) Les polymères répondant à la formule générale (VI) par exemple décrits dans le brevet français 1 400 366 : 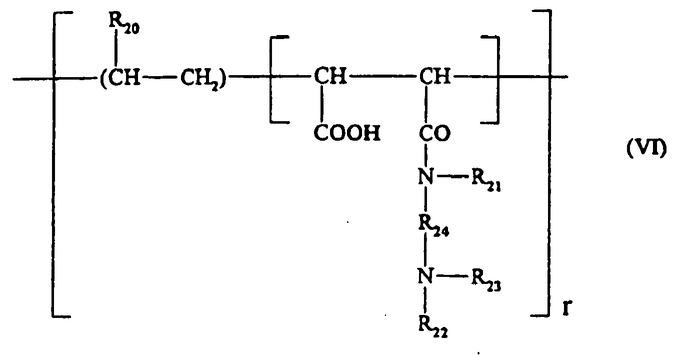 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)- , R₂₂ ayant les significations mentionnées ci-dessus,
      ainsi que les homologues supérieurs de ces radicaux et contenant jusqu'à 6 atomes de carbone.
   (8) Des polymères amphotères du type -D-X-D-X- choisis parmi:
      a) les polymères obtenus par action de l'acide chloracétique ou le chloracétate de sodium sur les composés comportant au moins un motif de formule :

         -D-X-D-X-D- (VII)

         où D désigne un radical 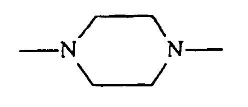 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
      b) Les polymères de formule :

         -D-X-D-X- (VII')
      où D désigne un radical 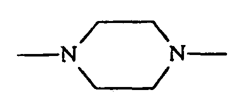 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
   (9) les copolymères alkyl(C1-C5)vinyléther / anhydride maléique modifié partiellement par semiamidification avec une N, N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères filmogènes amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple vendu sous la dénomination DIAFORMER Z301 par la société SANDOZ.

Les polymères filmogènes non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les copolymères de polyéthylène et d'anhydride maléïque;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
- les gommes de guar non ioniques chimiquement modifiées ou non modifiées. Les gommes de guar non ioniques non modifiées sont par exemple les produits vendus sous la dénomination VIDOGUM GH 175 par la société UNIPECTINE et sous la dénomination JAGUAR C par la société MEYHALL.
Les gommes de guar non-ioniques modifiées utilisables selon l'invention sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les radicaux alkyle des polymères non ioniques ont de 1 à 6 atomes de carbone sauf mention contraire.

Selon l'invention, on peut également utiliser les polymères filmogènes de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par:
a) 50 à 90% en poids d'acrylate de tertiobutyle ;
b) 0 à 40% en poids d'acide acrylique ;
c) 5 à 40% en poids de macromère siliconé de formule : 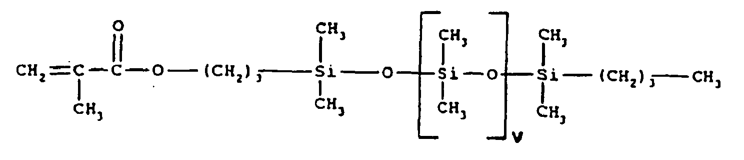 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser, comme polymères filmogènes (B), des polyuréthannes fonctionnalisés ou non, siliconés ou non, différents des polycondensats (A).

Le moyen de distribution, qui forme une partie du dispositif flacon pompe, est généralement constitué par au moins une pompe fonctionnant pour l'aspiration et/ou le refoulement du liquide et de l'air. Cette pompe est commandée par une tête de distribution, elle même comprenant une buse par laquelle la composition est vaporisée.

Selon l'invention, on utilise avantageusement un dispositif flacon pompe qui délivre une quantité de composition comprise entre 120 et 170 µl lorsque l'utilisateur applique 1 pression sur le bouton poussoir, et de préférence une quantité de composition comprise entre 140 et 160 µl.

Conformément à l'invention, le diamètre des gouttelettes est mesuré pour une température de la composition voisine de 20°C à l'intérieur du dispositif flacon pompe. En pratique, on place le dispositif flacon pompe à la température ambiante et on mesure le diamètre des gouttelettes à 20 cm de la buse.

Les polycondensats utilisés conformément à l'invention peuvent être solubles dans le milieu cosmétiquement acceptable ou encore former une dispersion dans ce milieu. La dispersion peut comprendre alors au moins 0,05 % de tensioactif permettant la mise en dispersion et le maintien en dispersion du polycondensat.

Selon l'invention, on peut utiliser tout type de tensioactif dans ladite dispersion, mais de préférence un tensioactif non ionique. La taille moyenne des particules du polycondensat dans la dispersion est de préférence comprise entre 0,1 et 1 micron.

La composition conforme à l'invention comprend avantageusement, en proportion relative en poids par rapport au poids total de la composition, entre 0,1 et 30 % du polycondensat (A) comprenant au moins une séquence polyuréthanne, plus avantageusement entre 0,5 et 20 %, et encore plus avantageusement entre 1 et 10 % de ce polycondensat.

La composition conforme à l'invention comprend avantageusement, en proportion relative en poids par rapport au poids total de la composition, entre 0,1 et 30 % du polymère filmogène (B), plus avantageusement entre 0,5 et 20 %, et encore plus avantageusement entre 1 et 10 % de polymère filmogène (B).

Elle peut comprendre un solvant organique additionnel, dans une proportion variant entre 0,5 et 80 %.

Conformément à l'invention, le solvant organique est notamment choisi dans le groupe comprenant les alcools en C₁ à C₄ tels que l'éthanol ou l'isopropanol, l'acétone, la méthyléthylcétone, l'acétate de méthyle, l'acétate de butyle, l'acétate d'éthyle, le diméthoxyéthane, le diéthoxyéthane et leurs mélanges. De manière préférentielle, on utilise l'éthanol.

Les compositions conformes à l'invention peuvent par ailleurs contenir des additifs cosmétiques conventionnels choisis notamment parmi les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères autres que ceux de l'invention, les silicones volatiles ou non, notamment les silicones anioniques, les polyols, les protéines et les vitamines.

## Revendications

1. Dispositif flacon pompe comprenant un réservoir contenant une composition capillaire ainsi que des moyens de distribution de la composition, **caractérisé par le fait que**:
(i) la composition comprend, dans un milieu cosmétiquement acceptable, au moins un polycondensat (A) comprenant au moins une séquence polyuréthanne et au moins un polymère filmogène (B) différent de (A); et
(ii) les polymères (A) et (B) et le dispositif étant choisis de sorte à obtenir, à la sortie du dispositif, des gouttelettes de composition de diamètre moyen Inférieur ou égal à 80 µm;
le polycondensat est formé par un arrangement de blocs, cet arrangement étant obtenu à partir de:
(1) au moins un composé qui contient deux ou plus de deux atomes d'hydro gène actifs par molécule;
(2) au moins un diol ou un mélange de diols contenant des radicaux acides ou leurs sels;
(3) au moins un di- ou polyisocyanate.
Et les composés (1) sont choisis dans le groupe comprenant les polyesterols:
le diamètre des gouttelettes est mesuré pour une température de la composition voisine de 20°C à l'intérieur du dispositif flacon pompe, et le diamètre des gouttelettes est mesuré à 20 cm de la buse, le polycondensat étant formé à partir d'au moins un composé supplémentaire ayant un squelette siliconé et choisi dans le groupe comprenant les polysiloxanes, les polyalkysiloxanes ou les polyarylsiloxanes notamment les polyéthylsiloxanes, les polyméthylsiloxanes et les polyphénylsiloxanes, comportant éventuellement des chaînes hydrocarbonées greffées sur les atomes de silicium.

2. Dispositif selon l'une quelconque des revendications précédentes, **caractérisé par le fait que** la composition contient un solvant organique.

3. Dispositif selon la revendication 1, **caractérisé par le fait que** le composé (2) est un acide carboxylique 2,2-hydroxyméthyl.

4. Dispositif selon la revendication 1, **caractérisé par le fait que** le composé (3) est choisi dans le groupe comprenant l'hexaméthylène diisocyanate, l'isophorondilsocyanate, le toluylendiisocyanate, le diphénylméthane 4,4'-dilsocyanate, le dicyclohexylméthane 4,4'-dlisocyanate, le méthylène-di-p-phényl diisocyanate, le méthylène-bis(4-cyclohexylisocyanate), l'isophorone diisocyanate, le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4- et de 2,6- toluène diisocyanate, le 2,2'-dichloro-4,4'-diisocyanato diphenylméthane, le 2,4-dibromo-1,5-diisocyanato naphtalène, le 1,4-diisocyanate butane, l'hexane-1,6-diisocyanate et le cyclohexane-1,4-diisocyanate.

5. Dispositif selon l'une quelconque des revendications précédentes, **caractérisé par le fait que** la composition comprend, en proportion relative en poids, entre 0,1 et 30 % du polycondensat (A), de préférence entre 0,5 et 20 %, et plus avantageusement encore entre 1 et 10 %.

6. Dispositif selon l'une quelconque des revendications précédentes, **caractérisé par le fait que** la composition comprend, en proportion relative en poids, entre 0,1 et 30 % du polymère filmogène (B), de préférence entre 0,5 et 20 %, et plus avantageusement encore entre 1 et 10 %.

7. Dispositif selon l'une quelconque des revendications précédentes, **caractérisé par le fait que** la composition comprend un solvant organique additionnel présent à une concentration rotative en poids comprise entre 0.5 et 80 %.

8. Dispositif selon l'une quelconque des revendication précédentes, **caractérisé par le fait que** le polymère filmogène (B) est un polymère anionique choisi parmi :
■ les polymères comportant des motifs carboxyliques dérivant de monomères mono ou diacides carboxyliques Insaturés de formule : 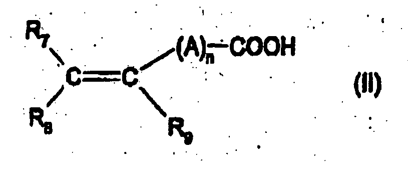
dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle;
- les polymères comprenant des motifs dérivant d'acide sulfonique tels que des motifs vinylsulfonique, styrènesulfonique, acrylamido alkylsulfonique.

9. Dispositif selon la revendication précédente, **caractérisé en ce que** le polymère filmogène anionique est choisi parmi :
- les copolymères d'acide acrylique tels que le terpolymère acide acryllque/acrylate d'éthyle/N-tertiobutylacrylamide :
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié :
- les copolymères d'acide méthacrylique et de méthacrylate de méthyle;
- le copolymère d'acide méthacrylique et d'acrylate d'éthyle;
- le copolymère acétate de vinyle/acide crotonique ;
- le terpolymère acétate de vinyle/acide crotonique/polyéthylèneglycol.

10. Dispositif selon l'une quelconque des revendications 1 à 7, **caractérisé en ce que** le polymère filmogène (B) est un polymère amphotère choisi parmi les polymères comportant des motifs dérivant
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

11. Dispositif selon l'une quelconque des revendications 1 à 7, **caractérisé par le fait que** le polymère filmogène (B) est un polymère non ionique choisi parmi:
- les polyalkyloxazolines;
- les homopolymères d'acétate de vinyle;
- les copolymères d'acétate de vinyle et d'ester acrylique;
- les copolymères d'acétate de vinyle et d'éthylène;
- les copolymères d'acétate de vinyle et d'ester maléïque;
- les copolymères de polyéthylène et d'anhydride maléïque;
- les homopolymères d'acrylates d'alkyle et les homopolymères de méthacrylates d'alkyle;
- les copolymères d'esters acryliques tels que par exemple les copolymères d'acrylates d'alkyle et de méthacrylates d'alkyle;
- les copolymères d'acrylonitrile et d'un monomère non ionique choisi par exemple parmi le butadiène et les (méth)acrylates d'alkyle; et
- les copolymères d'acrylate d'alkyle et d'uréthanne.

12. Dispositif selon l'une quelconque des revendications 1 à 7, **caractérisé par le fait que** le polymère filmogène (B) est un polymère cationique choisi parmi :
- le copolymère d'acrylamide et de diméthylaminoéthyl méthacrylate quaternisé au sulfate de diméthyle,
- les copolymères d'acrylamide et de chlorure de méthacryloyloxyéthyltriméthylammonium,
- le copolymère d'acrylamide et de méthosulfate de méthacryloyloxythyltriméthylammonium,
- les copolymères vinylpyrrolidone / acrylate ou méthacrylate de dialkylaminoalkyle quaternisés ou non,
- les terpolymères méthacrylate de diméthyl amino éthyle/ vinylcaprolactame/ vinylpyrrolidone,
- et le copolymère vinylpyrrolidone / méthacrylamide de diméthylaminopropyle quaternisé.

13. Dispositif selon l'une quelconque des revendications 1 à 7, **caractérisé par le fait que** le polymère filmogène (B) est un polymère siliconé greffe comprenant une portion polysiloxane et une portion constituée d'une chaîne organique non siliconée.

14. Dispositif selon l'une quelconque des revendications. 1 à 7, **caractérisé par le fait que** le polymère filmogène (B) est un polyuréthanne fonctionnalisé ou non, siliconé ou non différent de (A),

15. Dispositif selon l'une quelconque des revendications précédentes, **caractérisé par le fait qu'**il délivre une quantité de composition comprise entre 120 et 170 µl lorsque l'utilisateur applique 1 pression sur le bouton poussoir, et de préférence une quantité de composition comprise entre 140 et 160 µl.

16. Dispositif selon l'une quelconque des revendications précédentes, **caractérisé par le fait que** la composition contient en outre des additifs cosmétiques conventionnels choisis dans le groupe comprenant les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les silicones volatiles ou non, notamment les silicones anioniques, les polyols, les protéines et les vitamines.

17. Procédé capillaire pour la mise en forme ou le maintien de la coiffure, **Caractérisé par le fait qu'**il comprend la mise en oeuvre d'un dispositif conforme à l'une quelconque des revendications précédentes.

18. Utilisation d'un dispositif selon l'une quelconque des revendications 1 à 16 pour la fabrication d'un produit de coiffage.

## Claims

1. Pump-dispenser bottle device comprising a reservoir containing a hair composition, as well as means for distributing the composition, **characterized in that**:
(i) the composition comprises, in a cosmetically acceptable medium, at least one polycondensate (A) comprising at least one polyurethane block and at least one film-forming polymer (B) which is different from (A); and
(ii) the polymers (A) and (B) and the device being chosen so as to obtain, on leaving the device, droplets of composition with an average diameter of less than or equal to 80 µm;
the polycondensate is formed by an arrangement of blocks, this arrangement being obtained from:
(1) at least one compound which contains two or more than two active hydrogen atoms per molecule;
(2) at least one diol or a mixture of diols containing acid radicals or their salts;
(3) at least one di- or polyisocyanate
and the compounds (1) are chosen from the group comprising polyesterols;
the droplet diameter is measured for a composition temperature in the region of 20°C inside the pump-dispenser bottle device and the droplet diameter is measured 20 cm from the nozzle, the polycondensate being formed from at least one additional compound having a silicone skeleton, chosen from the group comprising polysiloxanes, polyalkylsiloxanes or polyarylsiloxanes, in particular polyethylsiloxanes, polymethylsiloxanes and polyphenylsiloxanes, optionally containing hydrocarbon-based chains grafted onto the silicon atoms.

2. Device according to Claim 1, **characterized in that** the composition contains an organic solvent.

3. Device according to Claim 1, **characterized in that** compound (2) is a 2,2-hydroxymethylcarboxylic acid.

4. Device according to Claim 1, **characterized in that** compound (3) is chosen from the group comprising hexamethylene diisocyanate, isophorone diisocyanate, toluylene diisocyanate, diphenylmethane 4,4'-diisocyanate, dicyclohexylmethane 4,4'-diisocyanate, methylenebis(p-phenyl) diisocyanate, methylenebis(4-cyclohexyl isocyanate), isophorone diisocyanate, toluene diisocyanate, 1,5-naphthalene diisocyanate, 4,4'-diphenylmethane diisocyanate, 2,2'-dimethyl-4,4'-diphenylmethane diisocyanate, 1,3-phenylene diisocyanate, 1,4-phenylene diisocyanate, mixtures of 2,4- and 2,6-toluene diisocyanate, 2,2'-dichloro-4,4'-diisocyanatodiphenylmethane, 2,4-dibromo-1,5-diisocyanatonaphthalene, butane 1,4-diisocyanate, 1,6-hexane diisocyanate and 1,4-cyclohexane diisocyanate.

5. Device according to any one of the preceding claims, **characterized in that** the composition comprises, as a relative proportion by weight, between 0.1 and 30% of the polycondensate (A), preferably between 0.5 and 20% and even more advantageously between 1 and 10%.

6. Device according to any one of the preceding claims, **characterized in that** the composition comprises, as a relative proportion by weight, between 0.1 and 30% of the film-forming polymer (B), preferably between 0.5 and 20% and even more advantageously between 1 and 10%.

7. Device according to any one of the preceding claims, **characterized in that** the composition comprises an additional organic solvent present in a relative weight concentration of between 0.5 and 80%.

8. Device according to any one of the preceding claims, **characterized in that** the film-forming polymer (B) is an anionic polymer chosen from:
- polymers comprising carboxylic units derived from unsaturated mono- or dicarboxylic acid monomers of formula: 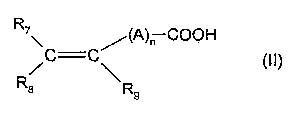 in which n is an integer from 0 to 10, A denotes a methylene group, optionally connected to the carbon atom of the unsaturated group, or to the neighbouring methylene group when n is greater than 1, via a heteroatom such as oxygen or sulphur, R₇ denotes a hydrogen atom or a phenyl or benzyl group, R₈ denotes a hydrogen atom or a lower alkyl or carboxyl group, R₉ denotes a hydrogen atom, a lower alkyl group or a -CH₂-COOH, phenyl or benzyl group;
- polymers comprising units derived from sulphonic acid, such as vinylsulphonic, styrenesulphonic or acrylamidoalkylsulphonic units.

9. Device according to the preceding claim, **characterized in that** the anionic film-forming polymer is chosen from:
- acrylic acid copolymers, such as acrylic acid/ethyl acrylate/N-tert-butylacrylamide terpolymer;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acids or anhydrides with vinyl esters, vinyl ethers, vinyl halides, phenylvinyl derivatives and acrylic acid and esters thereof, such as methyl vinyl ether/monoesterified maleic anhydride copolymers;
- copolymers of methacrylic acid and methyl methacrylate;
- the copolymer of methacrylic acid and ethyl acrylate;
- vinyl acetate/crotonic acid copolymer;
- vinyl acetate/crotonic acid/polyethylene glycol terpolymer.

10. Device according to any one of Claims 1 to 7, **characterized in that** the film-forming polymer (B) is an amphoteric polymer chosen from polymers comprising units derived from:
a) at least one monomer chosen from acrylamides and methacrylamides substituted on the nitrogen with an alkyl radical,
b) at least one acidic comonomer containing one or more reactive carboxylic groups, and
c) at least one basic comonomer such as esters containing primary, secondary, tertiary and quaternary amine substituents of acrylic and methacrylic acids and the product of quaternization of dimethylaminoethyl methacrylate with dimethyl or diethyl sulphate.

11. Device according to any one of Claims 1 to 7, **characterized in that** the film-forming polymer (B) is a nonionic polymer chosen from:
- polyalkyloxazolines;
- vinyl acetate homopolymers;
- copolymers of vinyl acetate and acrylic ester;
- copolymers of vinyl acetate and ethylene;
- copolymers of vinyl acetate and maleic ester;
- copolymers of polyethylene and maleic anhydride;
- alkyl acrylate homopolymers and alkyl methacrylate homopolymers;
- acrylic ester copolymers such as, for example, copolymers of alkyl acrylates and alkyl methacrylates;
- copolymers of acrylonitrile and a nonionic monomer chosen, for example, from butadiene and alkyl (meth)acrylates; and
- copolymers of alkyl acrylate and urethane.

12. Device according to any one of Claims 1 to 7, **characterized in that** the film-forming polymer (B) is a cationic polymer chosen from:
- the copolymer of acrylamide and dimethylaminoethyl methacrylate quaternized with dimethyl sulphate,
- copolymers of acrylamide and methacryloyloxyethyltrimethylammonium chloride,
- the copolymer of acrylamide and methacryloyloxyethyltrimethylammonium methosulphate,
- quaternized or non-quaternized vinylpyrrolidone/dialkylaminoalkyl acrylate or methacrylate copolymers,
- dimethylaminoethyl methacrylate/vinyl caprolactam/vinylpyrrolidone terpolymers, and
- vinylpyrrolidone/quaternized dimethylamino-propylmethacrylamide copolymer.

13. Device according to any one of Claims 1 to 7, **characterized in that** the film-forming polymer (B) is a grafted silicone polymer comprising a polysiloxane portion and a portion consisting of a non-silicone organic chain.

14. Device according to any one of Claims 1 to 7, **characterized in that** the film-forming polymer (B) is a functionalized or non-functionalized, silicone or non-silicone polyurethane which is different from (A).

15. Device according to any one of the preceding claims, **characterized in that** it delivers an amount of composition of between 120 and 170 µl when the user presses once on the push-button, and preferably an amount of composition of between 140 and 160 µl.

16. Device according to any one of the preceding claims, **characterized in that** the composition also contains conventional cosmetic additives chosen from the group comprising fatty substances, thickeners, softeners, antifoaming agents, moisturizers, antiperspirants, basifying agents, dyes, pigments, fragrances, preserving agents, surfactants, volatile or non-volatile silicones, in particular anionic silicones, polyols, proteins and vitamins.

17. Hair process for shaping or holding the hairstyle, **characterized in that** it comprises the use of a device in accordance with any one of the preceding claims.

18. Use of a device according to any one of Claims 1 to 16 for the manufacture of a hair styling product.

## Patentansprüche

1. Pumpflakon, der ein Reservoir mit einer Zusammensetzung für die Haarbehandlung und Einrichtungen zur Verteilung der Zusammensetzung umfasst und **dadurch gekennzeichnet ist, dass**:
(I) die Zusammensetzung in einem kosmetisch akzeptablen Medium mindestens ein Polykondensat (A), das mindestens eine Polyurethansequenz aufweist, und mindestens ein filmbildendes Polymer (B) enthält, das von (A) verschieden ist,
(II) die Polymere (A) und (B) und die Vorrichtung so gewählt sind, dass am Auslass der Vorrichtung Tröpfchen der Zusammensetzung mit einem mittleren Durchmesser von 80 µm oder darunter erhalten werden,
das Polykondensat aus einer Zusammenstellung von Blöcken besteht, wobei diese Anordnung ausgehend von den folgenden Verbindungen erhalten wird:
(1) mindestens einer Verbindung, die zwei oder mehr als zwei aktive Wasserstoffatome pro Molekül enthält,
(2) mindestens einem Diol oder einem Gemisch von Diolen, die Säuregruppen oder deren Salze enthalten, und
(3) mindestens einem Di- oder Polyisocyanat;
die Verbindungen (1) unter den Polyesterolen ausgewählt sind;
der Durchmesser der Tröpfchen bei einer Temperatur der Zusammensetzung in der Nähe von 20 °C im Innern des Pumpflakons und 20 cm von der Düse entfernt gemessen wird; wobei das Polykondensat ausgehend von mindestens einer zusätzlichen Verbindung gebildet wird, die ein Silicongerüst aufweist und unter den Polysiloxanen, Polyalkylsiloxanen oder Polyarylsiloxanen, insbesondere Polyethylsiloxanen, Polymethylsiloxanen und Polyphenylsiloxanen, ausgewählt ist, die gegebenenfalls auf die Siliciumatome gepfropfte Kohlenwasserstoffketten aufweisen.

2. Vorrichtung nach einem der vorhergehenden Ansprüche, **dadurch gekennzeichnet, dass** die Zusammensetzung ein organisches Lösungsmittel enthält.

3. Vorrichtung nach Anspruch 1, **dadurch gekennzeichnet, dass** es sich bei der Verbindung (2) um eine 2,2-Hydroxymethylcarbonsäure handelt.

4. Vorrichtung nach Anspruch 1, **dadurch gekennzeichnet, dass** die Verbindung (3) unter Hexamethylendiisocyanat, Isophorondiisocyanat, Toluylendiisocyanat, Diphenylmethan-4,4'-diisocyanat, Dicyclohexylmethan-4,4'-diisocyanat, Methylendi-p-phenyldiisocyanat, Methylen-bis(4-cyclohexylisocyanat), Isophorondiisocyanat, Toluoldiisocyanat, 1,5-Naphthalindiisocyanat, 4,4'-Diphenylmethan-diisocyanat, 2,2'-Dimethyl-4,4'-diphenylmethandiisocyanat, 1,3-Phenylendiisocyanat, 1,4-Phenylendiisocyanat, Gemischen von 2,4- und 2,6-Toluoldiisocyanat, 2,2'-Dichlor-4,4'-diisocyanato-diphenylmethan, 2,4-Dibrom-1,5-diisocyanatonaphthalin, 1,4-Diisocyanatobutan, Hexan-1,6-diisocyanat und Cyclohexan-1,4-diisocyanat ausgewählt ist.

5. Vorrichtung nach einem der vorhergehenden Ansprüche, **dadurch gekennzeichnet, dass** die Zusammensetzung das Polykondensat (A) in einer relativen Gewichtsmenge von 0,1 bis 30 %, vorzugsweise 0,5 bis 20 % und noch vorteilhafter 1 bis 10 % enthält.

6. Vorrichtung nach einem der vorhergehenden Ansprüche, **dadurch gekennzeichnet, dass** die Zusammensetzung das filmbildende Polymer (B) in einer relativen Gewichtsmenge von 0,1 bis 30 %, vorzugsweise 0,5 bis 20 % und noch vorteilhafter 1 bis 10 % enthält.

7. Vorrichtung nach einem der vorhergehenden Ansprüche, **dadurch gekennzeichnet, dass** die Zusammensetzung ein ergänzendes organisches Lösungsmittel enthält, das in einer relativen Gewichtsmenge von 0,5 bis 80 % enthalten ist.

8. Vorrichtung nach einem der vorhergehenden Ansprüche, **dadurch gekennzeichnet, dass** das filmbildende Polymer (B) ein anionisches Polymer ist, das ausgewählt ist unter:
- Polymeren mit Carbonsäureeinheiten, die von ungesättigten Mono- oder Dicarbonsäuremonomeren der folgenden Formel abgeleitet sind: 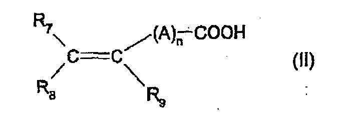 worin n 0 oder eine ganze Zahl von 1 bis 10 bedeutet, A eine Methylengruppe ist, die gegebenenfalls über ein Heteroatom, wie Sauerstoff oder Schwefel an das Kohlenstoffatom der ungesättigten Gruppe oder, wenn n größer 1 ist, an die benachbarte Methylengruppe gebunden ist, R₇ ein Wasserstoffatom, Phenyl oder Benzyl bedeutet, R₈ ein Wasserstoffatom, eine niedere Alkylgruppe oder Carboxy ist und R₉ ein Wasserstoffatom, eine niedere Alkylgruppe, die Gruppe -CH₂-COOH, Phenyl oder Benzyl bedeutet;
- Polymeren mit Einheiten, die von einer Sulfonsäure abgeleitet sind, wie Vinylsulfonsäureeinheiten, Styrolsulfonsäureeinheiten, Acrylamidoalkylsulfonsäureeinheiten.

9. Vorrichtung nach dem vorhergehenden Anspruch, **dadurch gekennzeichnet, dass** das anionische filmbildende Polymer ausgewählt ist unter:
- Copolymeren von Acrylsäure, wie dem Acrylsäure/Ethylacrylat/N-*t*-Butylacrylamid-Terpolymer;
- Copolymeren, die von Crotonsäure abgeleitet sind, wie den Vinylacetat/Vinyl-*t*-butylbenzoat/Crotonsäure-Terpolymeren und den Crotonsäure/Vinylacetat/Vinylneododecanoat-Terpolymeren;
- Polymeren, die von Maleinsäure oder Maleinsäureanhydrid, Fumarsäure oder Fumarsäureanhydrid, Itaconsäure oder Itaconsäureanhydrid und Vinylestern, Vinylethern, Vinylhalogeniden, Phenylvinylderivaten, Acrylsäure und ihren Estern abgeleitet sind, wie den Copolymeren Methylvinylether/monoverestertes Maleinsäureanhydrid;
- Methacrylsäure/Methylmethacrylat-Copolymeren;
- dem Methacrylsäure/Ethylacrylat-Copolymer;
- dem Vinylacetat/Crotonsäure-Copolymer; und
- dem Vinylacetat/Crotonsäure/Polyethylenglycol-Copolymer.

10. Vorrichtung nach einem der Ansprüche 1 bis 7, **dadurch gekennzeichnet, dass** das filmbildende Polymer (B) ein amphoteres Polymer ist, das unter den Polymeren mit Einheiten ausgewählt ist, die abgeleitet sind von:
a) mindestens einem Monomer, das unter den Acrylamiden oder Methacrylamiden ausgewählt ist, die am Stickstoffatom mit einer Alkylgruppe substituiert sind,
b) mindestens einem sauren Comonomer, das eine oder mehrere reaktive Carboxygruppen enthält, und
c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat.

11. Vorrichtung nach einem der Ansprüche 1 bis 7, **dadurch gekennzeichnet, dass** das filmbildende Polymer (B) ein nichtionisches Polymer ist, das ausgewählt ist unter:
- Polyalkyloxazolinen;
- Homopolymeren von Vinylacetat;
- Copolymeren von Vinylacetat und einem Acrylester;
- Copolymeren von Vinylacetat und Ethylen;
- Copolymeren von Vinylacetat und einem Maleinsäureester;
- Copolymeren von Polyethylen und Maleinsäureanhydrid;
- Homopolymeren von Alkylacrylaten und Homopolymeren von Alkylmethacrylaten;
- Copolymeren von Acrylestern, wie beispielsweise Copolymeren von Alkylacrylaten und Alkylmethacrylaten;
- Copolymeren von Acrylnitril und einem nichtionischen Monomer, das beispielsweise unter Butadien und Alkyl(meth)acrylaten ausgewählt ist;
- Copolymeren von Alkylacrylat und Urethan.

12. Vorrichtung nach einem der Ansprüche 1 bis 7, **dadurch gekennzeichnet, dass** das filmbildende Polymer (B) ein kationisches Polymer ist, das ausgewählt ist unter:
- dem Copolymer von Acrylamid und mit Dimethylsulfat quaternisiertem Dimethylaminoethylmethacrylat,
- Copolymeren von Acrylamid und Methacryloyloxyethyltrimethylammoniumchlorid,
- dem Copolymer von Acrylamid und Methacryloyloxyethyltrimethylammoniummethosulfat,
- Vinylpyrrolidon/Dialkylaminoalkylacrylat oder -methacrylat-Copolymere, die gegebenenfalls quaternisiert sind,
- Dimethylaminoethylmethacrylat/Vinylcaprolactam/Vinylpyrrolidon-Terpolymeren,
- und dem Vinylpyrrolidon/Dimethylaminopropylmethacrylamid-Copolymer, das quaternisiert ist.

13. Vorrichtung nach einem der Ansprüche 1 bis 7, **dadurch gekennzeichnet, dass** das filmbildende Polymer (B) ein gepfropftes Siliconpolymer ist, das einen Polysiloxanbereich und einen Bereich aufweist, der aus einer organischen, nicht siliconierten Kette besteht.

14. Vorrichtung nach einem der Ansprüche 1 bis 7, **dadurch gekennzeichnet, dass** das filmbildende Polymer (B) ein funktionalisiertes oder nicht funktionalisiertes, siliconiertes oder nicht siliconiertes Polyurethan ist, das von (A) verschieden ist.

15. Vorrichtung nach einem der vorhergehenden Ansprüche, **dadurch gekennzeichnet, dass** sie die Zusammensetzung in einer Menge von 120 bis 170 µl abgibt, wenn der Anwender einmal den Druckknopf betätigt, vorzugsweise in einer Menge von 140 bis 160 µl.

16. Vorrichtung nach einem der vorhergehenden Ansprüche, **dadurch gekennzeichnet, dass** die Zusammensetzung ferner herkömmliche kosmetische Zusatzstoffe enthält, die unter den Fettsubstanzen, Verdickungsmitteln, beruhigenden Stoffen, Schaumverhütungsmitteln, Hydratisierungsmitteln, Antiperspirantien, Alkalisierungsmitteln, Farbmitteln, Pigmenten, Parfums, Konservierungsmitteln, grenzflächenaktiven Stoffen, flüchtigen oder nichtflüchtigen Siliconen und insbesondere anionischen Siliconen, Polyolen, Proteinen und Vitaminen ausgewählt sind.

17. Verfahren zur Haarbehandlung für die Formgebung oder Festigung der Frisur, **dadurch gekennzeichnet, dass** eine Vorrichtung nach einem der vorhergehenden Ansprüche verwendet wird.

18. Verwendung einer Vorrichtung nach einem der Ansprüche 1 bis 16 für die Herstellung eines Produktes für die Haarbehandlung.
